# EUROPEAN PATENT SPECIFICATION

(11) **EP 1 418 174 B1**
(45) Date of publication and mention of the grant of the patent: **18.03.2009**
(21) Application number: 03029709.7
(22) Date of filing: 19.07.1999
(51) Int. Cl.: C07D 277/24, C07D 277/28, C07D 277/30, C07D 277/36, C07D 277/40, C07D 277/42, A61K 31/425

(54) **Amorphous Ritonavir**
Amorph ritonavir
Ritonavir Amorphe

(30) Priority: 20.07.1998 US 119345; 04.06.1999 US 326093
(43) Date of publication of application: 12.05.2004
(62) Divisional of application: 99934143.1
(73) Proprietor: ABBOTT LABORATORIES, Abbott Park, IL 60064 (US)
(72) Inventor: Bauer, John F., Lake Bluff, IL 60044 (US); Saleki-Gerhardt, Azita, Libertyville IL 60048 (US); Chemburkar, Sanjay R., Gurnee IL 60031 (US); Patel, Ketan, Arlington Heights IL60004 (US); Spiwek, Harry O., Kenosha WI 53142 (US)
(74) Representative: Modiano, Micaela Nadia

(56) References cited:
- US-A- 5 541 206
- US-A- 5 567 823
- US-A- 5 635 523
- US-A- 5 674 882

## Description

### Technical Field

This invention relates to an amorphous form of (2S,3S,5S)-5-(N-(N-((N-methyl-N-((2-isopropyl-4-thiazolyl)methyl)amino)-carbonyl)-L-valinyl)amino)-2-(N-((5-thiazolyl)methoxycarbonyl)amino)-1,6-diphenyl-3-hydroxyhexane and methods for its preparation

### Background of the Invention

Inhibitors of human immunodeficiency virus (HIV) protease have been approved for use in the treatment of HIV infection for several years. A particularly effective HIV protease inhibitor is (2S,3S,5S)-5-(N-(N-((N-methyl-N-((2-isopropyl-4-thiazolyl)methyl)amino)carbonyl)-L-valinyl)amino)-2-(N-((6-thiazolyl)-methoxycarbonyl)amino)-1,6-diphenyl-3-hydroxyhexane(ritonavir), which is marketed as NORVIR^{®}. Ritonavir is known to have utility for the inhibition of HIV protease, the inhibition of HIV infection, the inhibition of cytochrome P450 monooxygenase and the enhancement of the pharmacokinetics of compounds which are metabolized by cytochrome P450 monooxygenase. Ritonavir is particularly effective for the inhibition of HIV infection when used alone or in combination with one or more reverse transcriptase inhibitors and/or one or more other HIV protease inhibitors.

Ritonavir and processes for its preparation are disclosed in U.S. Patent No. 5,541,208, issued July 30, 1996. This patent discloses processes for preparing ritonavir which produce a crystalline polymorph of ritonavir which is termed crystalline Form I. The angular positions (two theta) of the characteristic peaks in the powder X-ray diffraction pattern of substantially pure Form I are 3.33° ± 0.1°, 6.76° ± 0.1°, 8.33° ± 0.1°, 14.61° ± 0.1°, 16.33° ± 0.1°, 16.76° ± 0.1°, 17.03° ± 0.1°, 18.02° ± 0.1°, 18.62° ± 0.1°, 19.47° ± 0.1°, 19.86° ± 0.1°, 20.25° ± 0.1°, 21.46° ± 0.1°, 23.46° ± 0.1° and 24.36° ± 0.1'.

Another process for the preparation of ritonavir is disclosed in U.S. Patent No. 5,567,823, issued October 22. 1996. The process disclosed in this patent also produces ritonavir as crystalline Form I.

Pharmaceutical compositions comprising ritonavir or a pharmaceutically acceptable salt thereof are disclosed in U.S. Patent Nos. 5,541,206, issued July 30, 1996; 5,484,801, issued January 16, 1996; 5,725,878, issued March 10, 1998; and 5,559,158, issued September 24, 1998 and in international Application No. WO98/22106, published May 28, 1998 (corresponding to U.S. Serial No. 08/966,495, filed November 7, 1997).

The use of ritonavir to inhibit an HIV infection is disclosed in U.S. Patent No. 5,541,206, issued July 30, 1996. The use of ritonavir in combination with one or more reverse transcriptase inhibitors to inhibit an HIV infection is disclosed in U.S. Patent No. 5,635,523, issued June 3, 1997. The use of ritonavir in combination with one or more HIV protease inhibitors to inhibit an HIV infection is disclosed in U.S. Patent No. 5,674,882, issued October 7, 1997. The use of ritonavir to inhibit cytochrome P450 monooxygenase and to enhance the pharmacokinetics of compounds metabolized by cytochrome P450 monooxygenase is disclosed in WO97/01349, published January 16, 1997 (corresponding to U.S. Serial No. 08/687,774, filed June 26, 1996).

It has now been unexpectedly discovered that ritonavir can be prepared as amorphous form.

All publications, issued patents and patent applications cited herein are hereby incorporated by reference.

### Brief Description of the Drawing

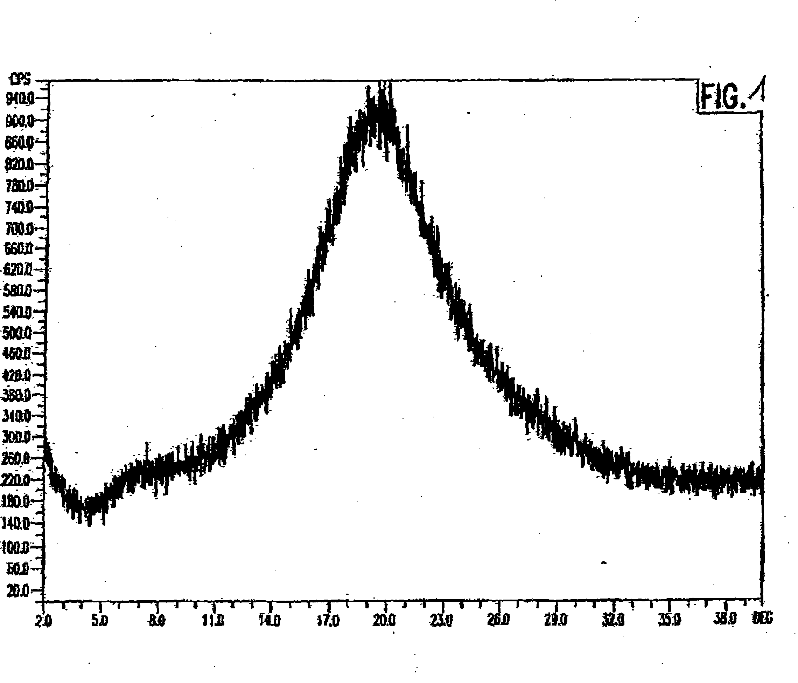

FIG. 1 is the powder X-ray diffraction pattern of substantially pure amorphous ritonavir.

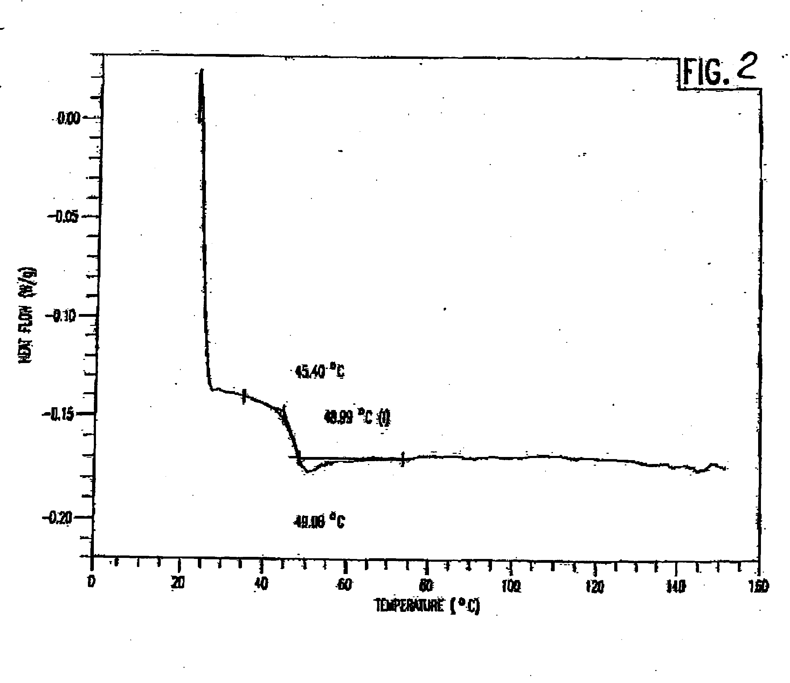

FIG. 2 is the differential scanning calorimetric thermogram for substantially pure amorphous ritonavir.

### Disclosure of the Invention

In accordance with the present invention, amorphous ritonavir in accordance with claim 1 has been prepared.

Substantially pure amorphous ritonavir is prepared from the Form I crystalline polymorph of ritonavir by melting Form I ritonavir and rapidly cooling the melt. Isolation of the resulting solid provides amorphous ritonavir.

Substantially pure amorphous ritonavir can also be prepared by slowly adding a solution of ritonavir Form I in a suitable solvent (methylene chloride and the like; preferably, methylene chloride) at a concentration of, preferably, about 1 g of ritonavir per about 1.5-2.0 mL of solvent (preferably, about 1 g of ritonavir/ about 1.5 mL of methylene chloride) to an anti-solvent (for example, hexane or heptane and the like; preferably, hexane) at a concentration of about 60-110 mL of antisolvent/ g of ritonavir, preferably, about 85-90 mL of hexane/ g of ritonavir, followed by isolation (for example, by filtration) of the resulting solid.

Similarly, substantially pure amorphous ritonavir can also be prepared by slowly adding a solution of ritonavir Form I in a suitable solvent such as methanol or the like at a concentration of, preferably, about 1 g of ritonavir per about 1.5-2.0 mL of solvent (preferably, about 1 g of ritonavir/ about 1.5 mL of methanol) to an anti-solvent such as methyl t-butyl ether (MTBE) or the like at a concentration of about 60-150 mL of antisolvent/ g of ritonavir, preferably, about 90-110 mL of MTBE/ g of ritonavir and, most preferably, about 100 mL of MTBE/ g of ritonavir, followed by isolation (for example, by filtration) of the resulting solid.

Substantially pure amorphous ritonavir can also be prepared by slowly adding a solution of ritonavir Form I in a suitable solvent (for example, methanol and the like; preferably, methanol) at a concentration of about 1 g of ritonavir per about 1.5-2.0 mL of solvent (preferably, about 1 g of ritonavir/ about 1.6 mL of methanol) to water at about 0°C at a concentration of about 400-500 mL of water/ g of ritonavir (preferably, about 400 mL of water/ g of ritonavir), followed by isolation (for example, by filtration) and drying of the resulting solid.

Substantially pure amorphous ritonavir can also be prepared by lyophilization of a solution ritonavir Form I. Preferred solvents are C1-C6 alcohols. A more preferred solvent is isobutanol.

The following examples will serve to further illustrate the preparation of the novel form of ritonavir of the invention.

### Example 1

### Preparation of Amorphous Ritonavir

Form I crystalline polymorph of ritonavir (100 g) was melted at 125°C by heating Form I. The melt was maintained at a temperature of 125°C for 3 hours. The melt was rapidly cooled by placing the container holding the melt into a Dewar flask containing liquid nitrogen. The resulting glass was ground with a mortar and pestle to provide amorphous ritonavir (100 g). Powder X-ray diffraction analysis confirmed that the product was amorphous. Differential scanning calorimetric analysis determined that the glass transition point was from about 46°C to about 49°C. (Measured onset at 45.4°C and which ends at 49.08°C, with a midpoint of 48.99°C).

### Comparative Example 2

### Preparation of Crystalline Ritonavir (Form II)

Amorphous ritonavir (40.0 g) was dissolved in boiling anhydrous ethanol (100 mL). Upon allowing this solution to cool to room temperature, a saturated solution was obtained. After standing overnight at room temperature, the resulting solid was isolated from the mixture by filtration and was air dried to provide Form II (approximately 24.0 g).

### Example 3

### Preparation of (2S)-N-((1S)-1-Benzyl-2-((4S,5S)-4-benzyl-2-oxo-1,3-oxazolidin-5-yl)ethyl)-2-((((2-isopropyl-1,3-thiazol-4-yl)methyl)amino)carbonyl)amino)-3-methylbutanamide

### Example 3a

### Preparation of (4S,5S)-5-((2S)-2-t-butyloxycarbonylamino-3-phenylpropyl)-4-benzyl)-1,3-oxazolidin-2-one

(2S,3S,5S)-2-Amino-3-hydroxy-5-t-butyloxycarbonylamino-1,6-diphenylhexane succinate salt (30 g, 63 mmol; U.S. Patent No. 5,654,466), ((5-thiazolyl)methyl)-(4-nitrophenyl)carbonate hydrochloride (22.2 g; U.S. Patent No. 5,597,926) and sodium bicarbonate (18.2 g) were mixed with 300mL of water and 300 mL of ethyl acetate and the mixture was stirred at room temperature for about 30 minutes. The organic layer was then separated and heated at about 60°C for 12 hours, and then stirred at 20-25°C for 6 hours. 3 mL of ammonium hydroxide (29% ammonia in water) was added and the mixture stirred for 1.5 hours. The resulting mixture was washed with 4 x 200 mL of 10% aqueous potassium carbonate and the organic layer was separated and evaporated under vacuum to provide an oil. The oil was suspended in about 250 mL of heptane. The heptane was evaporated under vacuum to provide a yellow solid. The yellow solid was dissolved in 300 mL of THF and 25 mL of 10% aqueous sodium hydroxide was added. After stirring for about 3 hours, the mixture was adjusted to pH 7 by addition of 4N HCl (about 16 mL). The THF was evaporated under vacuum to leave an aqueous residue, to which was added 300 mL of distilled water. After stirring this mixture, a fine suspension of solids resulted. The solid was collected by filtration and the filtered solid was washed with water (1400 mL) in several portions, resulting in the desired product.

### Example 3b

### Preparation of (4S,5S)-5-((2S)-2-amino-3-phenylpropyl)-4-benzyl-1,3-oxazolidin-2-one

The crude, wet product of Example 3a was slurried in 1N HCl (192 mL) and the slurry was heated to 70°C with stirring. After 1 hour, THF (100 mL) was added end stirring at 65°C was continued for 4 hours. The mixture was then allowed to cool to 20-25°C and was stirred overnight at 20-25°C. The THF was removed by evaporation under vacuum and the resulting aqueous solution was cooled to about 5°C, causing some precipitation to occur. The aqueous mixture was adjusted to pH 7 by addition of 50% aqueous sodium hydroxide (about 18.3 g). The resulting mixture was extracted with ethyl acetate (2 x 100 mL) at about 15°C. The combined organic extracts were washed with 100 mL of brine and the organic layer was separated and stirred with sodium sulfate (6 g) and Darco G-60 (3 g). This mixture was warmed on a hot plate for 1 hour at 45°C. The hot mixture was then filtered through a bed of diatomaceous earth and the filter pad was washed with ethyl acetate (100 mL). The filtrate was evaporated under vacuum to provide an oil. The oil was redissolved in methylene chloride (300 mL) and the solvent was evaporated under vacuum. The resulting oil was dried at room temperature under vacuum to provide the desired product (18.4 g) as a glassy syrup.

### Example 3c

### Preparation of (2S)-N-((1S)-1-Benzyl-2-((4S,5S)-4-benzyl-2-oxo-1,3-oxazolidin-5-yl)ethyl)-2-((((2-isopropyl-1,3-thiazol-4-yl)methyl)amino)carbonyl)amino)-3-methylbutanamide

N-((N-Methyl-N((2-isopropyl-4-thiazolyl)methyl)amino)carbonyl)-L-valine (10.6 g, 33.9 mmol; U.S. Patent No. 5,539,122 and International Patent Application No. WO96/00410), the product of Example 3b (10.0 g, 32.2 mmol) and 1-hydroxybenzotriazole (5.2 g, 34 mmol) were dissolved in THF (200 mL). 1,3-dicylcohexylcarbodiimide (DCC, 7.0 g, 34 mmol) was then added to the THF mixture and the mixture was stirred at 22°C for 4 hours. Citric acid (25 mL of 10% aqueous solution) was added and stirring continued for 30 minutes. The THF was then evaporated under vacuum. The residue was dissolved in ethyl acetate (250 mL) and washed with 10% citric acid solution (175 mL). NaCl (5 g) was added to accelerate the separation of the layers. The organic layer was sequentially washed with 10% aq. sodium carbonate (2 x 200 mL) and water (200 mL). The organic layer was then dried over sodium sulfate (20 g), filtered and evaporated under vacuum. The resulting product (20.7 g of a foam) was dissolved in not ethyl acetate (150 mL) and then heptane (75 mL) was added. Upon cooling, another 75 mL of heptane was added and the mixture was heated to reflux. Upon cooling to room temperature, no precipitate formed. The solvents were evaporated under vacuum and the residue was redissolved in a mixture of 200 mL ethyl acetate/100 mL heptane. The small amount of undissolved solid was removed by filtration. The filtrate was evaporated under vacuum and the residue was dissolved in a mixture of 100 mL ethyl acetate/ 50 mL heptane, giving a clear solution. The solution was cooled to -10°C and a white precipitate formed. The mixture was allowed to sit at -15°C for 24 hours. The resulting solid was collected by filtration, washed with 1:1 ethyl acetate/heptane (2 x 24 mL) and dried in a vacuum oven at 55°C to provide the deaired product as a beige solid (16.4 g).
¹H NMR (DMSO-d₆) δ 7.84 (1H, doublet J=8.6), 7.71 (1H, singlet), 7.32-7.11 (11H, mutiplat), 6.09 (1H, doublet J=8.5), 4.51 (1H AB J=16.2), 4.43 (1H AB J=16.2), 4.22 (1H, multiplet), 4.07 (1H, multiplet), 3.96 (1H, doublet of doublet J=7.3,7.4), 3.85 (1H, multiplet), 3.23 (1H, septuplet J=6.9), 2.89 (3H, singlet), 2.84-2.60 (4H, multiplet), 1.94 (1H, multiplet), 1.76-1.49 (2H, multiplet), 1.30 (6H, doublet J=6.9), 0.80 (3H, doublet J=5.8), 0.77 (3H, doublet J=5.8) ¹³C NMR (DMSO-d₆) δ 177.2, 171.6, 157.6, 157.5, 152.8, 138.3, 136.5, 129.5, 129.2, 128.2, 128.0, 128.4, 126.0, 114.0, 77.2, 58.9, 57.6, 46.2, 46.2, 40.4, 40.1, 39.1, 34.5, 32.4, 30.3, 22.8, 22.8, 19.4, 18.3.

### Comparative Example 4

### Preparation of Crystalline Ritonavir (Form II)

To a solution of 1.595 g of ritonavir Form I in 10 mL of 200 proof ethanol was added an amount of the product of Example 3c (approximately 50 micrograms) such that all of the added amount of the product of Example 3c did not dissolve. This mixture was allowed to stand at about 5°C for 24 hours. The resulting crystals were isolated by filtration through 0.45 micron nylon filter and air dried to provide ritonavir Form II.

### Comparative Example 5

### Alternative Preparation of Crystalline Ritonavir (Form II)

Ethyl acetate (6.0 L/kg of ritonavir) was added to ritonavir (Form I or a mixture of Form I and Form II) in a reaction vessel. The mixture was stirred and heated to 70°C until all solids were dissolved. The solution was filtered (utilizing a centrifuge pump and 5X20 inch cartridge filters having a porosity of 1.2 microns) and the filtrate was allowed to cool to 52°C at a rate of 2-10°C/hour. To this solution was added an amount of ritonavir Form II seed crystals (about 1.25 g of Form II seed crystals/kg of ritonavir) such that all of the seed crystals did not dissolve and the mixture was stirred at 52°C for not less than 1 hour at an agitation rate of 15 RPM. The mixture was then allowed to cool to 40°C at a rate of 10°C/hour. Heptane (2.8 L/kg of ritonavir) was added at a rate of 7L/minute with mixing. The mixture was allowed to cool to 25°C at a rate of 10°C/hour with mixing. Then the mixture was stirred for not less than 12 hours at 25°C. The product was isolated by filtration using a Heinkel type centrifuge (run time approximately 18 hours). The product was dried at 55°C under vacuum (50 mm Hg) for 16-25 hours to provide ritonavir crystal Form II.

### Example 6

### Preparation of Amorphous Ritonavir

Ritonavir Form I (40 g) was dissolved in methylene chloride (60 mL). This solution was slowly added over 15 minutes to a round bottom flask equipped with an overhead stirrer and containing hexanes (3,5 l.). The resulting slurry was allowed to stir for 10 minutes. The precipitate was filtered and dried at room temperature in a vacuum oven to provide amorphous ritonavir (40 g).

### Example 7

### Preparation of Amorphous Ritonavir

Ritonavir Form I (6 g) was dissolved in methanol (8 mL). This solution was slowly added to a round bottom flask equipped with an overhead stirrer and containing distilled water (2 L), while maintaining the internal temperature near 0°C. The resulting solid was filtered to give a sticky solid which was dried in a vacuum oven at 20-25°C for 12-18 hours to give amorphous ritonavir (2.5 g).

### Comparative Example 8

### Preparation of Ritonavir Form I

Ritonavir Form II (1 kg) was added to a reactor (A), followed by the addition of ethyl acetate (4 L). This mixture was refluxed until all of the solids were dissolved.

To a separate reactor (B) was added an amount of seed crystals of ritonavir Form I (5 g), followed by the addition of heptane (4 L), such that all of the seed crystals did not dissolve. This mixture (a slurry) was stirred at 23°C ±5°C.

The hot solution from reactor A was slowly filtered, using a 0.2 micron filter cartridge, into the mixture in reactor B over not less than 2 hours. The resulting slurry in reactor B was cooled to 20°C and stirred for not less than 3 hours. The resulting slurry was filtered, the filtered solid washed with heptane and than dried in a vacuum oven at 65°C to provide ritonavir Form I.

A preferred pharmaceutical composition comprising ritonavir, especially, ritonavir Form II, has the following composition, encapsulated in a soft elastic gelatin capsule.

| | |
|---|---|
| Ritonavir Form II | 100.0 mg |
| Ethanol, dehydrated | 120.0 mg |
| Oleic acid | 709.75 mg |
| Butylated hydroxytoluene | 0.25 mg |
| Polyoxyl 35 castor oil | 60.0 mg |
| (Cremophor EL^{®}) | |
| Water | 10.0 mg |

The preferred composition can be prepared according to the following method.

The following protocol is employed in the preparation of 1000 soft gelatin capsules:

| Scale (mg/capsule) | Name | Amount (g) |
|---|---|---|
| Q.S. | Nitrogen, N.F. | Q.S. |
| 118.0 | Ethanol, | 118.0 |
| | dehydrated, USP, 200 Proof | |
| 2.0 | Ethanol, | 2.0 |
| | dehydrated, USP, 200 Proof | |
| 0.25 | Butylated Hydroxytoluene, NF | 0.25 |
| 704.75 | Oleic Acid, NF | 704.75 |
| 100.0 | Ritonavir Form II | 100.0 |
| 10.0 | Water, purified, USP (distilled) | 10.0 |
| 60.0 | Polyoxyl 35 Castor Oil, NF | 60.0 |
| 5.000 | Oleic Acid, NF | 5.000 |

A mixing tank and suitable container are purged with nitrogen. 118.0 g of ethanol is weighed, blanketed with nitrogen, and held for later use. The second aliquot of ethanol (2 g) is then weighed, and mixed with 0.25 g of butylated hydroxytoluene until clear. The mixture is blanketed with nitrogen and held. The main mixing tank is heated to 28 °C (not to exceed 30 °C). 704.75 g of oleic acid is then charged into the mixing tank. 100.0 g of ritonavir Form II is then added to the oleic acid with mixing. The ethanol/butylated hydroxytoluene is then added to the mixing tank, followed by the 118.0 g of ethanol measured previously, and mixed for at least 10 minutes. 10 g of water is then charged into the tank and mixed until the solution is clear (for not less than 30 minutes). 60.0 g of Polyoxyl 35 castor oil is charged into the tank and mixed until uniform. The solution is stored at 2-8 °C until encapsulation. According to the procedures described in International Patent Application WO98/22106, 1.0 g of the solution is filled into each soft gelatin capsule and the soft gelatin capsules are then dried, and stored at 2-8 °C.

As used herein, the term "substantially pure", when used in reference to amorphous ritonavir, refers to amorphous ritonavir which is greater than about 90% pure. This means that the amorphous ritonavir does not contain more than about 10% of any other compound and, in particular, does not contain more than about 10% of any other form of ritonavir. More preferably, the term "substantially pure", when used in reference to amorphous ritonavir, refers to amorphous ritonavir which is greater than about 95% pure. This means that the amorphous ritonavir does not contain more than about 5% of any other compound and, in particular, does not contain more than about 5% of any other form of ritonavir. Even more preferably, the term "substantially pure", when used in reference to amorphous ritonavir, refers to amorphous ritonavir which is greater than about 97% pure. This means that the amorphous ritonavir does not contain more than about 3% of any other compound and, in particular, does not contain more than about 3% of any other form of ritonavir.

Powder X-ray diffraction analysis of samples was conducted in the following manner. Samples for X-ray diffraction analysis were prepared by spreading the sample powder (with no prior grinding required) in a thin layer on the sample holder and gently flattening the sample with a microscope slide. A Nicolet 12/V X-ray Diffraction System was used with the following parameters: X-ray source: Cu-Kα1; Range: 2.00-40.00° Two Theta; Scan Rate: 1.00 degree/minute; Step Size: 0.02 degrees; Wavelength: 1.540562 angstroms.

Characteristic powder X-ray diffraction pattern peak positions are reported for polymorphs in terms of the angular positions (two theta) with an allowable variability of ± 0.1°. This allowable variability is specified by the U.S. Pharmacopeia, pages 1843-1844 (1995). The variability of ± 0.1° is intended to be used when comparing two powder X-ray diffraction patterns. In practice, if a diffraction pattern peak from one pattern is assigned a range of angular positions (two theta) which is the measured peak position ± 0.1° and a diffraction pattern peak from the other pattern is assigned a range of angular positions (two theta) which is the measured peak position ± 0.1° and if those ranges of peak positions overlap, then de two peaks are considered to have the same angular position (two theta). For example, if a diffraction pattern peak from one pattern is determined to have a peak position of 5.20°, for comparison purposes the allowable variability allows the peak to be assigned a position in the range of 5.10°-5.30°. If a comparison peak from the other diffraction pattern is determined to have a peak position of 5.35°, for comparison purposes the allowable variability allows the peak to be assigned a position in the range of 5.25° - 5.45°. Because there is overlap between the two ranges of peak positions (i.e., 5.10° - 5.30° and 5.25° - 5.45°) the two peaks being compared are considered to have the same angular position (two theta).

Differential scanning calorimetric analysis of samples was conducted in the following manner. A T.A. Instruments Thermal Analyzer 3100 with Differential Scanning Calorimetry module 2910 was used, along with Modulated DSC software version 1.1A. The analysis parameters were: Sample weight 2.28 mg, placed in a covered, uncrimped aluminum pan; Heating rate: room temperature to 150°C at 5°C/minute under a nitrogen purge.

The foregoing is merely illustrative of the invention and is not intended to limit the invention to the disclosed embodiments. Variations and changes which are obvious to one skilled in the art are intended to be within the scope and nature of the invention which are defined in the appended claims.

## Claims

1. Amorphous ritonavir, containing no more than 10% of any other form of ritonavir, **characterised by** the DSC thermogram recorded at a heating rate of 5°C/min and a glass transition temperature from about 45°C to about 49° C.

2. A process for the preparation of the compound of claim 1 comprising adding a solution of ritonavir to an antisolvent.

3. A process for the preparation of the compound of claim 1 comprising adding a solution of ritonavir in methylene chloride to hexane.

4. A process for the preparation of the compound of claim 1 comprising adding a solution of ritonavir Form I in methylene chloride at a concentration of about 1 g of ritonavir per about 1.5-2.0 mL of methylene chloride to hexane at a concentration of about 60-110 mL of hexane per gram of ritonavir.

5. A process for the preparation of the compound of claim 1 comprising adding a solution of ritonavir Form I in methylene chloride at a concentration of about 1 g of ritonavir per about 1.5 mL of methylene chloride to hexane at a concentration of about 85-90 mL of hexane per gram of ritonavir.

6. A process for the preparation of the compound of claim 1 comprising adding a solution of ritonavir in methanol to methyl t-butyl ether.

7. A process for the preparation of the compound of claim 1 comprising adding a solution of ritonavir Form I in methanol at a concentration of about 1 g of ritonavir per about 1.5-2.0 mL of methanol to hexane at a concentration of about 60-150 mL of hexane per gram of ritonavir.

8. A process for the preparation of the compound of claim 1 comprising adding a solution of ritonavir Form I in methanol at a concentration of about 1 g of ritonavir per about 1.5 mL of methanol to hexane at a concentration of about 90-110 mL of hexane per gram of ritonavir.

9. A process for the preparation of the compound of claim 1 comprising adding a solution of ritonavir in methanol to water.

10. A process for the preparation of the compound of claim 1 comprising adding a solution of ritonavir Form I in methanol at a concentration of about 1 g of ritonavir per about 1.5-2.0 mL of methanol to water at a concentration of about 400-500 mL of water per gram of ritonavir.

11. A process for the preparation of the compound of claim 1 comprising adding a solution of ritonavir Form I in methanol at a concentration of about 1 g of ritonavir per about 1.6 mL of methanol to water at a concentration of about 400 mL of water per gram of ritonavir.

12. A process for the preparation of the compound of claim 1 comprising lyophilization of a solution of ritonavir.

13. A process for the preparation of the compound of claim 1 comprising lyophilization of a solution of ritonavir in isobutanol.

14. A pharmaceutical composition comprising amorphous ritonavir according to claim 1.

15. The pharmaceutical composition according to claim 14, wherein the amorphous ritonavir contains no more than 5% of any other form of ritonavir.

16. The pharmaceutical composition according to claim 15, wherein the amorphous ritonavir contains no more than 3% of any other form of ritonavir.

17. Amorphous ritonavir according to claim 1 as a protease inhibitor.

18. Use of amorphous ritonavir according to claim 1 for manufacturing a medicament.

19. Use of amorphous ritonavir according to claim 1 for manufacturing a medicament for treating a HIV infection.

20. Amorphous ritonavir according to claim 1, prepared according to the process of any one of claims 2-13.

## Patentansprüche

1. Amorphes Ritonavir, das nicht mehr als 10% irgendeiner anderen Form von Ritonavir enthält, **gekennzeichnet durch** das DSC-Thermogramm, aufgenommen bei einer Erwärmungsgeschwindigkeit von 5°C/min, und eine Glasübergangstemperatur von ungefähr 45°C bis ungefähr 49°C.

2. Verfahren zur Herstellung der Verbindung gemäß Anspruch 1, welches das Hinzufügen einer Lösung von Ritonavir zu einem Anti-Lösungsmittel umfasst.

3. Verfahren zur Herstellung der Verbindung gemäß Anspruch 1, welches das Hinzufügen einer Lösung von Ritonavir in Methylenchlorid zu Hexan umfasst.

4. Verfahren zur Herstellung der Verbindung gemäß Anspruch 1, welches das Hinzufügen einer Lösung von Ritonavir Form I in Methylenchlorid bei einer Konzentration von ungefähr 1 g Ritonavir pro ungefähr 1,5-2,0 ml Methylenchlorid zu Hexan bei einer Konzentration von ungefähr 60-110 ml Hexan pro Gramm Ritonavir umfasst.

5. Verfahren zur Herstellung der Verbindung gemäß Anspruch 1, welches das Hinzufügen einer Lösung von Ritonavir Form I in Methylenchlorid bei einer Konzentration von ungefähr 1 g Ritonavir pro ungefähr 1,5 ml Methylenchlorid zu Hexan bei einer Konzentration von ungefähr 85-90 ml Hexan pro Gramm Ritonavir umfasst.

6. Verfahren zur Herstellung der Verbindung gemäß Anspruch 1, welches das Hinzufügen einer Lösung von Ritonavir in Methanol zu Methyl-t-butylether umfasst.

7. Verfahren zur Herstellung der Verbindung gemäß Anspruch 1, welches das Hinzufügen einer Lösung von Ritonavir Form I in Methanol bei einer Konzentration von ungefähr 1 g Ritonavir pro ungefähr 1,5-2,0 ml Methanol zu Hexan bei einer Konzentration von ungefähr 60-150 ml Hexan pro Gramm Ritonavir umfasst.

8. Verfahren zur Herstellung der Verbindung gemäß Anspruch 1, welches das Hinzufügen einer Lösung von Ritonavir Form I in Methanol bei einer Konzentration von ungefähr 1 g Ritonavir pro ungefähr 1,5 ml Methanol zu Hexan bei einer Konzentration von ungefähr 90-110 ml Hexan pro Gramm Ritonavir umfaßt.

9. Verfahren zur Herstellung der Verbindung gemäß Anspruch 1, welches das Hinzufügen einer Lösung von Ritonavir in Methanol zu Wasser umfasst.

10. Verfahren zur Herstellung der Verbindung gemäß Anspruch 1, welches das Hinzufügen einer Lösung von Ritonavir Form I in Methanol bei einer Konzentration von ungefähr 1 g Ritonavir pro ungefähr 1,5-2,0 ml Methanol zu Wasser bei einer Konzentration von ungefähr 400-500 ml Wasser pro Gramm Ritonavir umfasst.

11. Verfahren zur Herstellung der Verbindung gemäß Anspruch 1, welches das Hinzufügen einer Lösung von Ritonavir Form I in Methanol bei einer Konzentration von ungefähr 1 g Ritonavir pro ungefähr 1,6 ml Methanol zu Wasser bei einer Konzentration von ungefähr 400 ml Wasser pro Gramm Ritonavir umfasst.

12. Verfahren zur Herstellung der Verbindung gemäß Anspruch 1, welches die Lyophilisation einer Lösung von Ritonavir umfasst.

13. Verfahren zur Herstellung der Verbindung gemäß Anspruch 1, welches die Lyophilisation einer Lösung von Ritonavir in Isobutanol umfasst.

14. Pharmazeutische Zusammensetzung, die amorphes Ritonavir gemäß Anspruch 1 umfasst.

15. Pharmazeutische Zusammensetzung gemäß Anspruch 14, worin das amorphe Ritonavir nicht mehr als 5% irgendeiner anderen Form von Ritonavir enthält.

16. Pharmazeutische Zusammensetzung gemäß Anspruch 15, worin das amorphe Ritonavir nicht mehr als 3% irgendeiner anderen Form von Ritonavir enthält.

17. Amorphes Ritonavir gemäß Anspruch 1 als Proteaseinhibitor.

18. Verwendung von amorphem Ritonavir gemäß Anspruch 1 zur Herstellung eines Medikaments.

19. Verwendung von amorphem Ritonavir gemäß Anspruch 1 zur Herstellung eines Medikaments zur Behandlung einer HIV-Infektion.

20. Amorphes Ritonavir gemäß Anspruch 1, hergestellt gemäß dem Verfahren eines beliebigen der Ansprüche 2-13.

## Revendications

1. Ritonavir amorphe contenant au plus 10 % de toute autre forme de ritonavir, **caractérisé par** le thermogramme DSC enregistré à une vitesse de chauffage de 5°C/min et une température de transition vitreuse d'environ 45°C à environ 49°C.

2. Procédé pour la préparation du composé selon la revendication 1 comprenant l'addition d'une solution de ritonavir à un antisolvant.

3. Procédé pour la préparation du composé selon la revendication 1 comprenant l'addition d'une solution de ritonavir dans du chlorure de méthylène à de l'hexane.

4. Procédé pour la préparation du composé selon la revendication 1 comprenant l'addition d'une solution de forme I de ritonavir dans du chlorure de méthylène à une concentration d'environ 1 g de ritonavir pour environ 1,5-2,0 ml de chlorure de méthylène à de l'hexane à une concentration d'environ 60-100 ml d'hexane par gramme de ritonavir.

5. Procédé pour la préparation du composé selon la revendication 1 comprenant l'addition d'une solution de forme I de ritonavir dans du chlorure de méthylène à une concentration d'environ 1 g de ritonavir pour environ 1,5 ml de chlorure de méthylène à de l'hexane à une concentration d'environ 85-90 ml d'hexane par gramme de ritonavir.

6. Procédé pour la préparation du composé selon la revendication 1 comprenant l'addition d'une solution de ritonavir dans du méthanol à du méthyl-t-tubyléther.

7. Procédé pour la préparation du composé selon la revendication 1 comprenant l'addition d'une solution de forme I de ritonavir dans du méthanol à une concentration d'environ 1 g de ritonavir pour environ 1,5-2,0 ml de méthanol à de l'hexane à une concentration d'environ 60-150 ml d'hexane par gramme de ritonavir.

8. Procédé pour la préparation du composé selon la revendication 1 comprenant l'addition d'une solution de forme I de ritonavir dans du méthanol à une concentration d'environ 1 g de ritonavir pour environ 1,5 ml de méthanol à de l'hexane à une concentration d'environ 90-110 ml d'hexane par gramme de ritonavir.

9. Procédé pour la préparation du composé selon la revendication 1 comprenant l'addition d'une solution de ritonavir dans du méthanol à de l'eau.

10. Procédé pour la préparation du composé selon la revendication 1 comprenant l'addition d'une solution de forme I de ritonavir dans du méthanol à une concentration d'environ 1 g de ritonavir pour environ 1,5-2,0 ml de méthanol à de l'eau à une concentration d'environ 400-500 ml d'eau par gramme de ritonavir.

11. Procédé pour la préparation du composé selon la revendication 1 comprenant l'addition d'une solution de forme I de ritonavir dans du méthanol à une concentration d'environ 1 g de ritonavir pour environ 1,6 ml de méthanol à de l'eau à une concentration d'environ 400 ml d'eau par gramme de ritonavir.

12. Procédé pour la préparation du composé selon la revendication 1 comprenant la lyophilisation d'une solution de ritonavir.

13. Procédé pour la préparation du composé selon la revendication 1 comprenant la lyophilisation d'une solution de ritonavir dans de l'isobutanol.

14. Composition pharmaceutique comprenant du ritonavir amorphe selon la revendication 1.

15. Composition pharmaceutique selon la revendication 14, dans laquelle le ritonavir amorphe contient au plus 5 % de toute autre forme de ritonavir.

16. Composition pharmaceutique selon la revendication 15, dans laquelle le ritonavir amorphe contient au plus 3 % de toute autre forme de ritonavir.

17. Ritonavir amorphe selon la revendication 1 comme inhibiteur de protéase.

18. Utilisation de ritonavir amorphe selon la revendication 1 pour la fabrication d'un médicament.

19. Utilisation de ritonavir amorphe selon la revendication 1 pour la fabrication d'un médicament destiné au traitement d'une infection VIH.

20. Ritonavir amorphe selon la revendication 1 préparé selon le procédé selon l'une quelconque des revendications 2-13.
